# EUROPEAN PATENT SPECIFICATION

(11) **EP 0 787 463 B1**
(45) Date of publication and mention of the grant of the patent: **01.10.2003**
(21) Application number: 97300543.2
(22) Date of filing: 29.01.1997
(51) Int. Cl.: A61B 5/022

(54) **Oscillometric type electronic sphygmomanometer**
Elektronisches Sphygmomanometer vom oszillometrischen Typus
Sphygmomanomètre électronique utilisant la méthode oscillométrique

(30) Priority: 30.01.1996 JP 1400796
(43) Date of publication of application: 06.08.1997
(73) Proprietor: Citizen Watch Co. Ltd., Tokyo 188-8511 (JP)
(72) Inventor: Okamoto, Makoto, Tanashi-shi, Tokyo (JP)
(74) Representative: Hackney, Nigel John

(56) References cited:
- EP-A- 0 208 520
- EP-A- 0 733 339
- US-A- 5 170 795
- US-A- 5 542 428

## Description

The present invention relates to a sphygmomanometer, particularly to an oscillometric type electronic sphygmomanometer which compresses an artery by inflating a cuff, detects pulse wave amplitude during deflation of the inflated cuff, and determines blood pressure using the pulse wave amplitude.

Conventional sphygmomanometers utilizing Korotkoff's sounds as biological sounds are complicated in structure. Also, because the Korotkoff's sounds are very weak, these cannot be exactly detected when a microphone is not precisely placed related to blood vessels. Further, when the Korotkoff's sounds of a patient are weak, these cannot be readily detected. There have been difficulties in detecting blood pressure using the conventional sphygmomanometers.

For these reasons, so-called oscillometric type electronic sphygmomanometers using a cuff have recently been developed. With these oscillometric type electronic sphygmomanometers, the blood pressure can be detected by detecting pulse wave amplitude generated and diminished in the cuff during deflation of the inflated cuff, transforming the pulse wave amplitude to electronic signals, and extracting specific pulse wave amplitude from the electronic signals to determine the blood pressure.

When these oscillometric type electronic sphygmomanometers are used, the cuff is wrapped around the brachium of a patient. The pressure in the cuff is then raised to compress the brachium for arterial occlusion and the pressure in the cuff is detected as signals during deflation of the inflated cuff. Components of pulse wave included in the signals of the detected pressure are detected using a means for detecting pulse wave amplitude. The information for the components of the pulse wave is then transformed to a series of pulse wave amplitude. The blood pressure is determined based on the maximum value among the series of amplitude and also the cuff pressure. Specifically, the cuff pressure corresponding to the maximum pulse wave amplitude is determined as the mean blood pressure. The cuff pressure corresponding to the pulse wave amplitude of the high pressure side equivalent to 50% of the maximum pulse wave amplitude is determined as the maximal blood pressure. Also, the cuff pressure corresponding to the pulse wave amplitude of the low pressure side equivalent to 70% of the maximum pulse wave amplitude is determined as the minimal blood pressure.

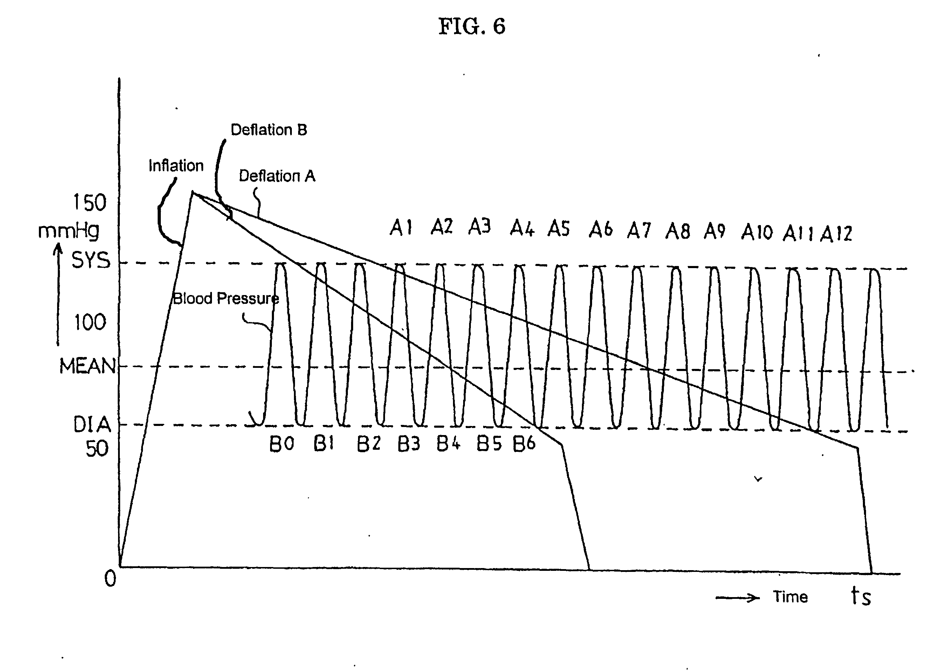

When gradually reducing cuff pressure using a cuff pressure control means after binding the brachium of a patient with the cuff of the oscillometric type electronic sphygmomanometer to compress an artery, and raising the cuff pressure to 150-200 mmHg in the same manner as in common practice, the following pattern is observed in the fluctuation of a pulse wave generated and diminished in the cuff in relation to the change in pulse pressure and cuff pressure to be provided. The pulse wave amplitude is suddenly increased close to the maximal blood pressure of the patient and the rate of decrease in the pulse wave amplitude is suddenly reduced close to the minimal blood pressure. Figure 6 is a graphic chart showing the relation between the cuff pressure applied to the cuff and the blood pressure.

In Figure 6, SYS, DIA, and MEAN on the ordinate show values of the maximal blood pressure, minimal blood pressure, and mean blood pressure respectively. As mentioned above, when a cuff pressure raised to 150-200 mmHg is reduced at the rate of 3 mmHg/sec which is normal pumping speed, the values of cuff pressure are memorized at intervals of 3 mmHg when the number of pulses of the patient is 60 BPM (Beat Per Min).

In Figure 6, for example, the deflation A shows a case where the exhaust speed is 2 mmHg/sec and the deflation B shows a case where the exhaust speed is 5 mmHg. In the case of the deflation A in Fig. 6, cuff pressure for detecting each pulse wave amplitude is shown as A1, A2, ...A12. In the case of the deflation B, cuff pressure for detecting each pulse wave amplitude is similarly shown as B1, B2, ... B12. Compared with the number of pulses in the deflation A, that in the deflation B is reduced by half.

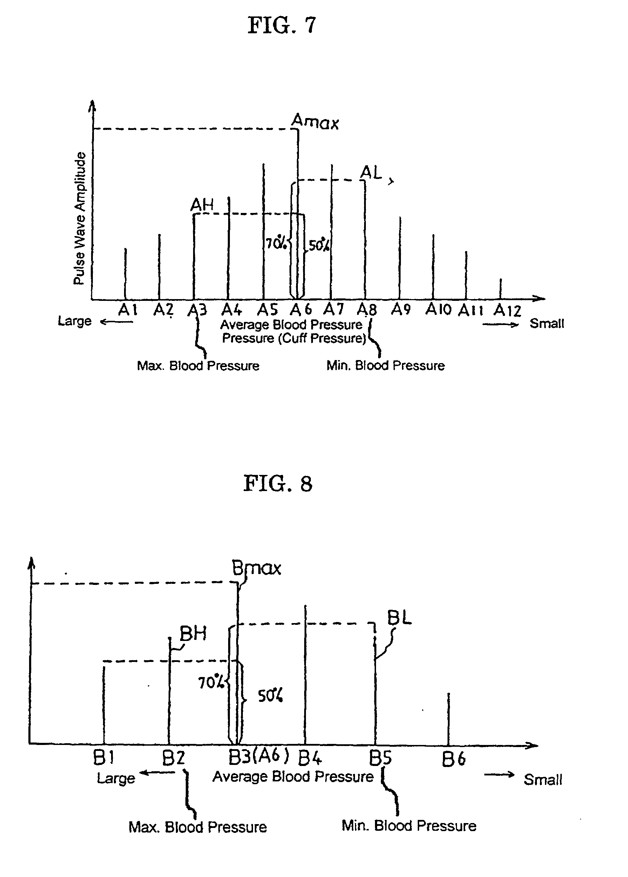

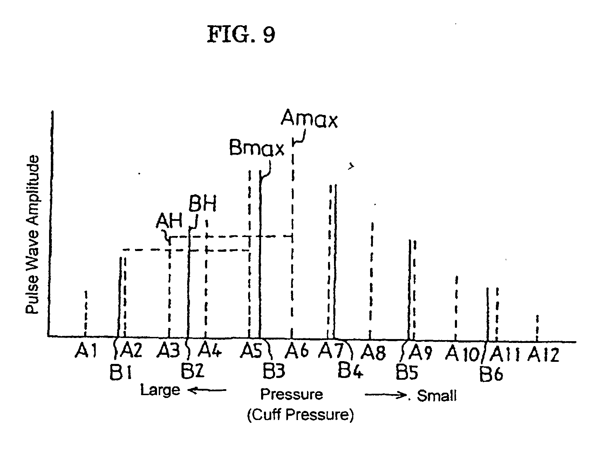

Figure 7 and Figure 8 are graphic charts showing the relation between pulse wave amplitude and pressure (cuff pressure) in the cases of the deflations A and B respectively. Figure 9 is a graphic chart formed by overlapping Figure 7 and Figure 8.

As shown in Figure 7, in the case of the deflation A in which the exhaust speed is 2 mmHg/sec, cuff pressure is detected at the same interval of 2 mmHg. In Figure 7, the cuff pressure A6 corresponding to the maximum pulse wave amplitude Amax is determined as the mean blood pressure, the cuff pressure A3 corresponding to the pulse wave amplitude AH of the high pressure side corresponding to 50% of the maximum pulse wave amplitude Amax is determined as the maximal blood pressure, and the cuff pressure A8 corresponding to the pulse wave amplitude AL of the low pressure side corresponding to 70% of the maximum pulse wave amplitude Amax is determined as the minimal blood pressure.

In the case of the deflation A, where the deflation is carried out at a low rate, the maximal and minimal blood pressures can be measured with comparatively high accuracy. However, because the deflation is slow, the artery of the patient is compressed for a long period of time, which causes discomfort to the patient.

If the rate of the deflation is increased according to the deflation B shown in Fig. 8 to reduce the time of compression of the artery, the cuff pressure B3 corresponding to the maximum pulse wave amplitude Bmax is positioned between the cuff pressures A5 and A6 in the deflation A. Therefore, the maximum amplitude of pulse wave Bmax becomes lower than the maximum pulse wave amplitude Amax (a value of the pulse wave amplitude at the cuff pressure A6).

However, there are problems in an oscillometric sphygmomanometer improved to reduce time for compressing the artery by increasing the rate of deflation so that the patient is free from the discomfort. Specifically, the maximum pulse wave amplitude Bmax is lower than the maximum pulse wave amplitude Amax in the deflation A as shown in Fig. 8 and Fig. 9. Hence, the cuff pressure B2 corresponding to the pulse wave amplitude BH which initially exceeds the pulse wave amplitude of the high pressure side equivalent to 50% of the maximum pulse wave amplitude Bmax is temporarily determined as the maximal blood pressure (the maximal blood pressure A3 in the deflation A is between the cuff pressures B1 and B2 in the deflation B). Also, the cuff pressure B5 corresponding to the pulse wave amplitude BL which is initially lower than the pulse wave amplitude of the low pressure side equivalent to 70% of the maximum pulse wave amplitude Bmax is temporarily determined as the minimal blood pressure (the minimal blood pressure A8 in the deflation A is between the cuff pressures B4 and B5 in the deflation B). Therefore, in the deflation B which is intended to release the patient from the measurement of blood pressure as soon as possible by increasing the exhaust speed, there are the drawbacks that the accuracy of measurement is extremely low compared with that in the deflation A, leading to incorrect measurements. Also, when the deflation of a cuff pressure control means is increased to the rate shown in the deflation B, differences in the circumference of the brachium of a patient and in the temperature often cause incorrect measurement.

The present invention has been achieved in view of this situation and preferably aims to provide an oscillometric type electronic sphygmomanometer capable of calculating a true maximum pulse wave amplitude corresponding to an accurate mean blood pressure, while deflation is accelerated to release the patient as soon as possible from the discomfort caused by compression of the artery. Also, the present invention preferably aims to provide an oscillometric type electronic sphygmomanometer capable of determining the maximal and minimal pressures by calculating the pulse wave amplitude corresponding to maximal or minimal pressure based on the true maximum pulse wave amplitude.

The oscillometric type electronic sphygmomanometer of the present invention is defined in claim 1 the features of the preamble being known from US-5 170 795.

Preferred embodiments are described in dependent claims 2-5.

As explained above, the present invention satisfies the demands of subjects (patients) who want to minimize the discomfort caused by compression on the artery. Also, the present invention provides a highly accurate electronic sphygmomanometer. Specifically, the present invention accurately determines the maximal and minimal blood pressures in the case where the detected number of pulse wave is reduced when the deflation is accelerated. The blood pressure can be exactly measured corresponding to change in deflation and the number of pulse wave. Therefore, the present invention provides a highly reliable oscillometric type electronic sphygmomanometer.
Figure 1 is a block diagram showing a major part of an embodiment of the oscillometric type electronic sphygmomanometer.
Figure 2 is a block diagram of a means for determining blood pressure corresponding to Figure 1.
Figure 3 is a block diagram showing an overall view of the embodiment of the oscillometric type electronic sphygmomanometer.
Figure 4 is a graph showing the relation between a pulse wave amplitude and pressure, in the embodiment.
Figure 5 is an enlarged sectional chart of the high pressure side corresponding to Figure 4.
Figure 6 is a graph showing the relation between cuff pressure and blood pressure for explaining the difference between the prior art and the present invention.
Figure 7 is a graph showing the relation between pulse wave amplitude and pressure in the deflation A corresponding to Figure 6.
Figure 8 is a graph showing the relation between pulse wave amplitude and pressure in the deflation B corresponding to Figure 6.
Figure 9 is a graph showing the relation between pulse wave amplitude and pressure by overlapping Fig. 7 and Fig. 8.

In these Figures, the following symbols for indicating various parts are applied.
1: Cuff
2: Cuff pressure control means
3: Cuff pressure detecting means
4: Pulse wave amplitude detecting means
5: Variation curve calculating means
6: Maximum pulse wave determining means
7: Blood pressure determining means
7a: First standard level determining means
7b: Regression straight line calculating means
7c: Second standard level determining means
7d: Blood pressure calculating means
8: Inflation-deflation section
9: Pressure inspection section
10: Microcomputer
11: Operating switch
12: Liquid crystal display
13: Buzzer
C: Variation curve
D: Regression straight line
Cmax: True maximum pulse wave amplitude
Bmax: Measured maximum pulse wave amplitude
SYS: Maximal blood pressure
DIA: Minimal blood pressure

The present invention will be explained in detail by way of an embodiment of the oscillometric type electronic sphygmomanometer of the present invention with reference to the drawings.

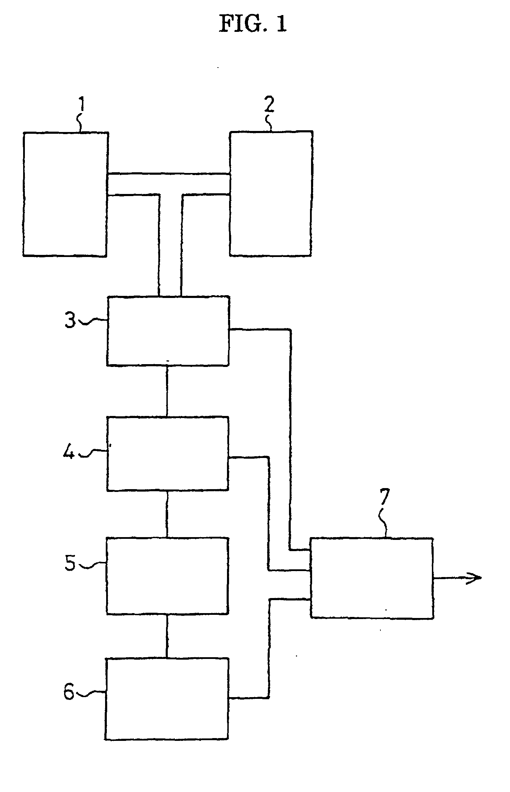

With reference to Figure 1, a major part of the embodiment of the oscillometric type electronic sphygmomanometer of the present invention is explained.

A cuff 1 is used to compress the artery of a subject. A control means 2 for controlling cuff pressure is connected with the cuff 1 and used to inflate and deflate the cuff 1 to a fixed pressure. A means 3 for detecting cuff pressure is connected with the cuff 1 and the control means 2 and used to detect pressure in the cuff 1. A means 4 for detecting pulse wave amplitude is used to detect a pulse wave amplitude output during the deflation of the cuff 1 using the means 3 for detecting cuff pressure. A means 5 for calculating a variation curve is used to calculate a variation curve for pulse wave amplitude based on the maximum pulse wave amplitude measured and detected by the means 4 for detecting pulse wave amplitude and the pulse wave amplitudes before and after the maximum pulse wave amplitude. A means 6 for determining the maximum pulse wave is used to determine, as the true maximum pulse wave amplitude, a pulse wave amplitude corresponding to the maximum value on the variation curve calculated by the means 5 for calculating a variation curve.

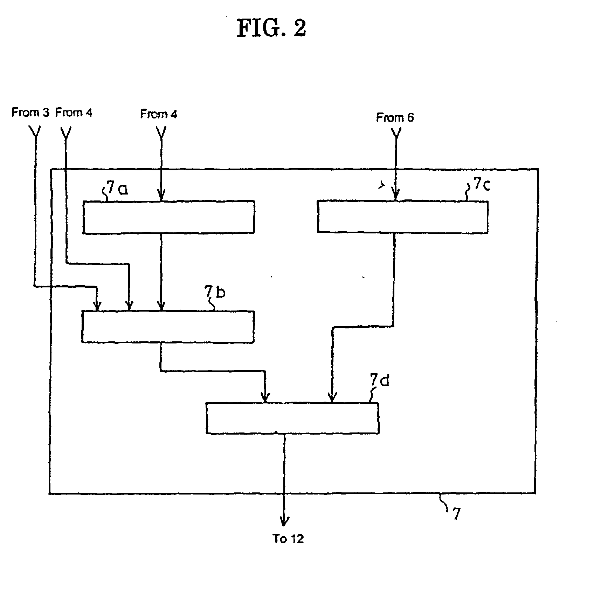

A means 7 for determining the blood pressure, as shown in Figure 2, consists of a means 7a for determining a first standard level based on the maximum pulse wave amplitude measured by the means 4 for detecting pulse wave amplitude, a means 7b for calculating a regression straight line indicating the ascent and descent of the pulse wave based on the variations of pulse wave amplitude detected before and after the pulse wave amplitudes detected based on the first standard level, a means 7c for determining a second standard level based on the true maximum pulse wave amplitude determined by the means 6 for determining the maximum pulse wave, and a means 7d for determining the maximal and minimal pressures based on pulse wave amplitudes on the regression straight line corresponding to the second standard level. The blood pressure calculated by the means 7d for calculating blood pressure is displayed on a liquid crystal display 12 described below.

In the present embodiment, the functions of the means 4 for calculating pulse wave amplitude, the means 5 for calculating the variation curve, the means 6 for determining the maximum pulse wave, and the means 7 for determining the blood pressure are included in a program stored in a microcomputer 10 illustrated below.

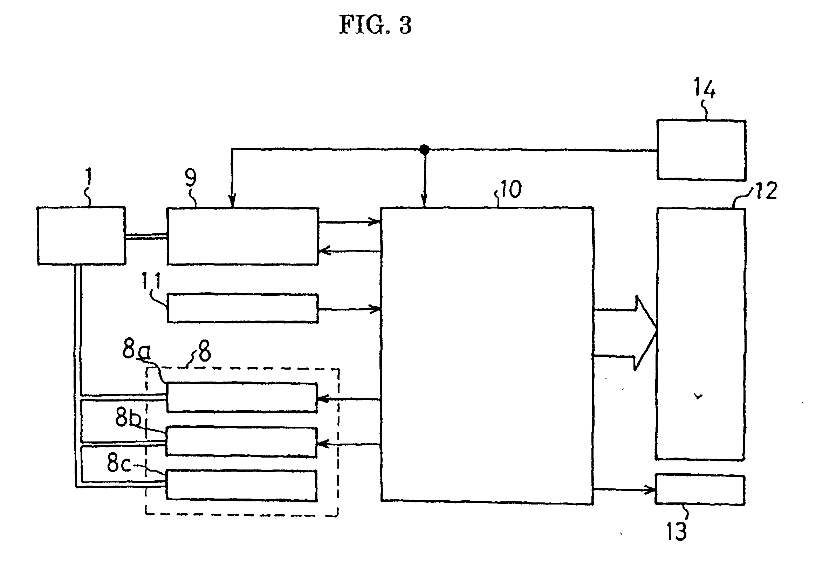

With reference to Figure 3, the actions and functions of the embodiment of the oscillometric type electronic sphygmomanometer will be explained.

In actual use, all segments of the liquid crystal 12 are displayed, by turning on a power supply which is inactivated until blood pressure measurement is commenced, the ring of a buzzer 13 and a cell 14 are checked, pressure of the cuff 1 is measured, and a measurement system is checked. Also, the pressure to be provided by a pressure setting system is set.

Next, the brachium of a patient is bound with the cuff 1. After actuating the power switch of an operating switch 11 illustrated below, operation of an air supply facility is started by actuating a measurement switch to start pressurizing the cuff 1 by supplying air to the cuff 1 using a pump 8a positioned in an inflation-deflation section 8. At this time, after inflating the cuff 1 to, for example, 150-200 mmHg by actuating a pressure setting switch, the cuff 1 is gradually deflated at a comparatively high rate, for example, 5 mm Hg/sec using an automatic exhaust valve 8c. An electromagnetic valve 8b releases exhaust air from the cuff 1 to restore the cuff to atmospheric pressure.

Next, pressure signals measured by a pressure sensor are converted to frequencies using an A/D conversion circuit of a pressure inspection section 9 included in the means 3 for detecting cuff pressure. The converted frequencies are input to a microcomputer 10, which controls the operation of the entire system of the electronic sphygmomanometer and also calculates the maximal blood pressure SYS and the minimal blood pressure DIA from the pressure signals detected by the pressure inspection section 9 using a program based on an oscillometric method.

Here, the oscillometric method utilizes a pulse wave amplitude generated in cuff pressure by the pulsation of the heart when the pressure of the cuff 1 is gradually reduced. This pulse wave amplitude is increased when the cuff pressure is close to the maximal blood pressure, maximized at the mean blood pressure, and becomes approximately constant from close to the minimal blood pressure. With the oscillometric method, therefore, analog signals output from the pressure inspection section 9 are converted to digital signals and the maximal and minimal blood pressures are determined after extracting a specific pulse wave amplitude. After the measurement of the blood pressure is finished in this manner, the results are displayed on the liquid crystal display 12 and the subject is advised of the completion of the blood pressure measurement by means of the buzzer 13. The cell 14 includes a plurality (for example four) of size AA dry batteries.

The operating switch 11 consists of a power supply switch for starting and stopping the electronic sphygmomanometer system, a measurement switch for starting the air supply facility which pressurizes the cuff 1, a memory switch for calling up the last results of measured maximal and minimal blood pressures, a pressure setting switch for setting the pressure to be provided by the air supply facility.

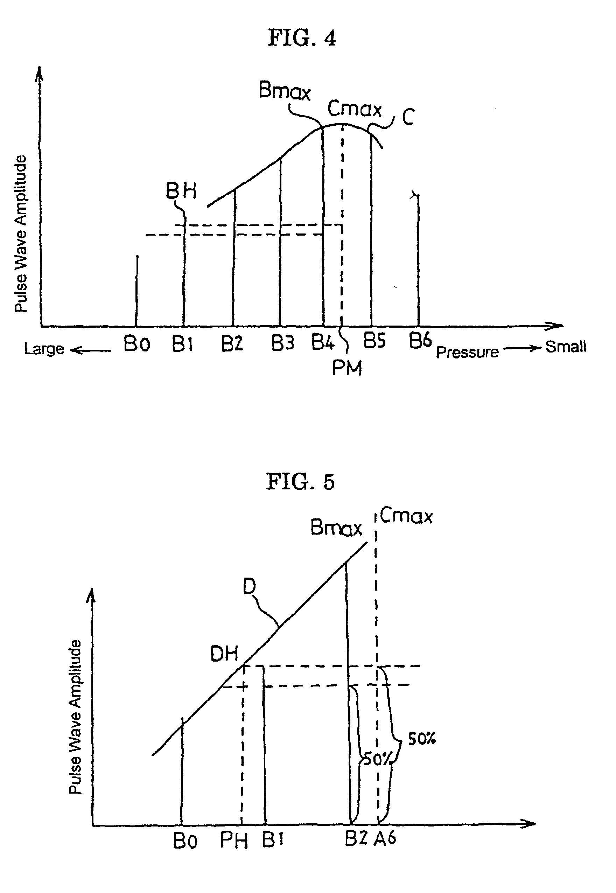

Figure 4 is a graph showing the relation between pulse wave amplitude and pressure in the present embodiment in the case where pressure is reduced at a rate of 5 mmHg/sec in accordance with the deflation B shown in Figure 6. In the electronic sphygmomanometer of this embodiment, as shown in Figure 4, the relation between pulse wave amplitude and pressure is calculated and plotted as a variation curve C by the means 5 for calculating variation curve by means of the least square method and the maximum value Cmax on the variation curve C is determined as the true maximum pulse wave amplitude Cmax. The cuff pressure PM corresponding to the true maximum pulse wave amplitude Cmax is the mean blood pressure.

Figure 5 is an enlarged sectional chart in a range of pressure higher than the cuff pressure PM shown in Figure 4. A method for calculating the maximal blood pressure SYS will be explained with reference to Figure 5.

At first, the cuff pressure B1 corresponding to the pulse wave amplitude which initially exceeds 50% of the maximum pulse wave amplitude Bmax calculated by the means 7a based on the measured maximum pulse wave amplitude Bmax is determined as temporary maximal blood pressure. The regression straight line D plotted for cuff pressure versus pulse wave amplitude indicating ascent of the pulse wave is calculated by the means 7b based on cuff pressures B0 and B2 corresponding to the pulse wave amplitudes before and after the cuff pressure B1. Next, the cuff pressure PH corresponding to the pulse wave amplitude DH on the regression straight line D corresponding to 50% of the maximum pulse wave amplitude Cmax determined by means 7c based on the true maximum pulse wave amplitude Cmax is determined as the maximal blood pressure SYS by the means 7d for calculating blood pressure.

The minimal blood pressure DIA is determined in the same manner. Specifically, although there is no reference diagram, a regression curve plotted for cuff pressure versus pulse wave amplitude indicating the descent of the pulse wave amplitude is calculated. Next, based on the true maximum pulse wave amplitude Cmax, the cuff pressure corresponding to the pulse wave amplitude on the regression curve D corresponding to 70% of the maximum pulse wave amplitude Cmax is determined as the minimal blood pressure DIA by the means 7d for calculating blood pressure.

With the oscillometric type electronic sphygmomanometer of the present embodiment, an air supply facility for supplying air to the brachium is provided, the cuff is pressurized higher than the estimated maximal blood pressure, a variable curve is calculated based on the maximum pulse wave amplitude measured while the cuff is gradually deflated at a fixed rate by an automatic exhaust facility, and the pulse wave amplitudes detected before and after the maximum pulse wave amplitude, and a pulse wave amplitude corresponding to the peak of the variable curve are calculated as the true maximum pulse wave amplitude. In addition, a first standard level is determined by the means 7 for determining blood pressure based on the measured maximum pulse wave amplitude, a regression straight line indicating the ascent or descent of the pulse wave is calculated from the pulse wave amplitude detected based on the first standard level and pulse wave amplitudes detected before and after the pulse wave amplitude, and, based on the true maximum pulse amplitude, the maximal and minimal blood pressures are calculated from the pulse wave amplitude on the regression straight line corresponding to a second standard level to display the results on the liquid crystal display 12.

Specifically, the oscillometric type electronic sphygmomanometer of the present invention is not limited to the above embodiment and may include many variable embodiments. For example, the means 4 for detecting pulse wave amplitude, means 5 for calculating variable curve, means 6 for determining maximum pulse waves, and means 7 for determining blood pressure may be devices constituted with an electronic circuit instead of using programs. Also, the cuff pressure for determining the maximum and minimal blood pressures may be pulse wave amplitudes on the regression straight line corresponding to ratios other than 50% or 70% of the maximum pulse wave amplitude Cmax.

## Claims

1. An oscillometric type electronic sphygmomanometer comprising:
a cuff for compressing the artery of a subject;
a control means for controlling inflation or deflation of the cuff;
a means for detecting pressure in the cuff; and
a means for detecting pulse wave amplitude in the course of controlling the cuff pressure by the control means;
**characterised in that** the sphygmomanometer comprises:
a means for calculating a variation curve from a maximum pulse wave amplitude measured by the means for detecting pulse wave amplitude and pulse wave amplitudes detected before and after the measured maximum pulse wave amplitude;
a means for determining, as true maximum pulse wave amplitude, the pulse wave amplitude corresponding to a maximum value on the variation curve; and
a means for determining at least the maximal or minimal blood pressure using the true maximum pulse wave amplitude.

2. The oscillometric type electronic sphygmomanometer according to Claim 1, wherein the means for determining at least the maximal or minimal blood pressure comprises:
a means for determining a first standard level as a pulse wave amplitude of a predetermined ratio of the measured maximum pulse wave amplitude;
a means for specifying a measured pulse wave amplitude in an increase trend of the pulse wave on the basis of the first standard level, and calculating a regression straight line indicating the increase of the pulse wave from pulse wave amplitudes and cuff pressures detected before and after the specified measured pulse wave amplitude;
a means for determining a second standard level as a pulse wave amplitude of a predetermined ratio of the true maximum pulse wave amplitude; and
a means for calculating the maximal blood pressure from the pulse wave amplitude on the increasing regression straight line corresponding to the second standard level.

3. The oscillometric type electronic sphygmomanometer according to Claim 1, wherein the means for determining at least the maximal or minimal blood pressure comprises:
a means for determining a first standard level as a pulse wave amplitude of a predetermined ratio of the measured maximum pulse wave amplitude;
a means for specifying a measured pulse wave amplitude in a decrease trend of the pulse wave on the basis of the first standard level, and calculating a regression straight line indicating the decrease of the pulse wave from pulse wave amplitudes and cuff pressures detected before and after the specified measured pulse wave amplitude;
a means for determining a second standard level as a pulse wave amplitude of a predetermined ratio of the true maximum pulse wave amplitude; and
a means for calculating the minimal blood pressure from the pulse wave amplitude on the decreasing regression straight line corresponding to the second standard level.

4. The oscillometric type electronic sphygmomanometer according to Claim 2, wherein the second standard level is a pulse wave amplitude equal to 50% of the true maximum pulse wave amplitude, and the means for calculating the maximal blood pressure determines, as the maximal blood pressure, the cuff pressure on the regression straight line corresponding to a pulse wave amplitude equal to the second standard level.

5. The oscillometric type electronic sphygmomanometer according to Claim 3, wherein the second standard level is a pulse wave amplitude equal to 70% of the true maximum pulse wave amplitude, and the means for calculating the minimal blood pressure determines, as the minimal blood pressure, the cuff pressure on the regression straight line corresponding to a pulse wave amplitude equal to the second standard level.

## Patentansprüche

1. Elektronisches Sphygmomanometer vom oszillometrischen Typus umfassend:
eine Manschette zum Zusammendrücken der Arterie einer Person;
ein Steuerungsmittel zum Steuern des Druckaufbaus oder Druckabbaus in der Manschette;
ein Mittel zum Detektieren des Drucks in der Manschette; und
ein Mittel zum Detektieren der Pulswellenamplitude im Zuge des Steuerns des Manschettendrucks durch das Steuerungsmittel;
**dadurch gekennzeichnet, dass** das Sphygmomanometer Folgendes umfasst:
ein Mittel, um eine Abweichungskurve aus einer maximalen Pulswellenamplitude, die durch das Mittel zum Detektieren der Pulswellenamplitude gemessen wurde, und Pulswellenamplituden, die vor und nach dem gemessenen Maximalpuls detektiert wurden, zu berechnen;
ein Mittel, um als wahre maximale Pulswellenamplitude die einem Maximalwert auf der Abweichungskurve entsprechende Pulswellenamplitude zu bestimmen; und
ein Mittel, um anhand der wahren maximalen Pulswellenamplitude zumindest den maximalen oder minimalen Blutdruck zu bestimmen.

2. Elektronisches Sphygmomanometer vom oszillometrischen Typus nach Anspruch 1, worin das Mittel zum Bestimmen zumindest des maximalen oder minimalen Blutdrucks Folgendes umfasst:
ein Mittel zum Bestimmen eines ersten Standardwerts als eine Pulswellenamplitude eines vorbestimmten Anteils der gemessenen maximalen Pulswellenamplitude;
ein Mittel zum Spezifizieren einer gemessenen Pulswellenamplitude in einem Anstiegstrend der Pulswelle basierend auf dem ersten Standardwert, und zum Berechnen einer Regressionsgerade, die den Anstieg der Pulswelle anzeigt, die aus vor und nach der spezifizierten gemessenen Pulswellenamplitude detektierten Pulswellenamplituden und Manschettendruckwerten ermittelt wird;
ein Mittel zum Bestimmen eines zweiten Standardwerts als eine Pulwellenamplitude eines vorbestimmten Anteils der wahren maximalen Pulswellenamplitude; und
ein Mittel zum Berechnen des maximalen Blutdrucks aus der Pulswellenamplitude auf der ansteigenden Regressionsgeraden, die dem zweiten Standardwert entspricht.

3. Elektronisches Sphygmomanometer vom oszillometrischen Typus nach Anspruch 1, worin das Mittel zum Bestimmen zumindest des maximalen oder minimalen Blutdrucks Folgendes umfasst:
ein Mittel zum Bestimmen eines ersten Standardwerts als eine Pulswellenamplitude eines vorbestimmten Anteils der gemessenen maximalen Pulswellenamplitude;
ein Mittel zum Spezifizieren einer gemessenen Pulswellenamplitude in einem Abstiegstrend der Pulswelle auf Basis des ersten Standardwerts, und zum Berechnen einer Regressionsgerade, die den Abfall der Pulswelle anzeigt, die aus vor und nach der spezifizierten gemessenen Pulswellenamplitude detektierten Pulswellenamplituden und Manschettendruckwerten ermittelt wird;
ein Mittel zum Bestimmen eines zweiten Standardwerts als eine Pulswellenamplitude eines vorbestimmten Anteils der wahren maximalen Pulswellenamplitude; und
ein Mittel zum Berechnen des minimalen Blutdrucks aus der Pulswellenamplitude auf der abfallenden Regressionsgeraden, die dem zweiten Standardwert entspricht.

4. Elektronisches Sphygmomanometer vom oszillometrischen Typus nach Anspruch 2, worin der zweite Standardwert eine Pulswellenamplitude ist, die 50 % der wahren maximalen Pulswellenamplitude entspricht, und das Mittel zum Berechnen des maximalen Blutdrucks als maximalen Blutdruck den Manschettendruck auf der Regressionsgeraden annimmt, der einer dem zweiten Standardwert entsprechenden Pulswellenamplitude entspricht.

5. Elektronisches Sphygmomanometer vom oszillometrischen Typus nach Anspruch 3, worin der zweite Standardwert eine Pulswellenamplitude ist, die 70 % der wahren maximalen Pulswellenamplitude beträgt, und das Mittel zum Berechnen des minimalen Blutdrucks als minimalen Blutdruck den Manschettendruck auf der Regressionsgeraden annimmt, der einer dem zweiten Standardwert entsprechenden Pulswellenamplitude entspricht.

## Revendications

1. Tensiomètre électronique du type oscillométrique comprenant:
un brassard pour comprimer l'artère d'un sujet;
un moyen de commande pour commander le gonflement ou le dégonflement du brassard;
un moyen pour détecter la pression dans le brassard;et
un moyen pour détecter l'amplitude de l'onde impulsionnelle au cours de la commande de la pression du brassard par le moyen de commande;
**caractérisé en ce que** le tensiomètre comprend:
un moyen pour calculer une courbe de variation à partir d'une amplitude d'onde impulsionnelle maximum mesurée par le moyen pour détecter l'amplitude de l'onde impulsionnelle et des amplitudes d'ondes impulsionnelles détectées avant et après l'amplitude d'onde impulsionnelle maximum mesurée;
un moyen pour déterminer, comme amplitude d'onde impulsionnelle maximum vraie, l'amplitude de l'onde impulsionnelle correspondant à une valeur maximum sur la courbe de variation; et
un moyen pour déterminer au moins la pression sanguine maximale ou minimale en utilisant l'amplitude d'onde impulsionnelle maximum vraie.

2. Tensiomètre électronique du type oscillométrique selon la revendication 1, où le moyen pour déterminer au moins la pression sanguine maximale ou minimale comprend:
un moyen pour déterminer un premier niveau standard comme une amplitude d'onde impulsionnelle d'un rapport prédéterminé de l'amplitude d'onde impulsionnelle maximum mesurée;
un moyen pour spécifier une amplitude d'onde impulsionnelle mesurée lors d'une tendance à l'augmentation de l'onde impulsionnelle sur la base du premier niveau standard, et pour calculer une ligne de régression droite indiquant l'augmentation de l'onde impulsionnelle à partir des amplitudes d'ondes impulsionnelles et des pressions de brassard détectées avant et après l'amplitude de l'onde impulsionnelle mesurée spécifiée;
un moyen pour déterminer un second niveau standard comme amplitude de l'onde impulsionnelle d'un rapport prédéterminé de l'amplitude d'onde impulsionnelle maximum vraie; et
un moyen pour calculer la pression sanguine maximale à partir de l'amplitude de l'onde impulsionnelle sur la ligne droite d'augmentation de la régression correspondant au deuxième niveau standard.

3. Tensiomètre électronique du type oscillométrique selon la revendication 1, où le moyen pour déterminer au moins la pression sanguine maximale ou minimale comprend:
un moyen pour déterminer un premier niveau standard comme une amplitude de l'onde impulsionnelle d'un rapport prédéterminé de l'amplitude de l'onde impulsionnelle maximum mesurée;
un moyen pour spécifier une amplitude mesurée de l'onde impulsionnelle lors d'une tendance à la diminution de l'onde impulsionnelle sur la base du premier niveau standard, et pour calculer une ligne droite de régression indiquant la diminution de l'onde impulsionnelle des amplitudes d'ondes impulsionnelles et des pressions de brassard détectées avant et après l'amplitude mesurée spécifiée de l'onde impulsionnelle;
un moyen pour déterminer un deuxième niveau standard comme une amplitude de l'onde impulsionnelle d'un rapport prédéterminé de l'amplitude d'onde impulsionnelle maximum vraie; et
un moyen pour calculer la pression sanguine minimale à partir de l'amplitude de l'onde impulsionnelle sur la ligne droite de régression diminuante correspondant au deuxième niveau standard.

4. Tensiomètre électronique du type oscillométrique selon la revendication 2, où le deuxième niveau standard est une amplitude de l'onde impulsionnelle égale à 50% de l'amplitude maximum vraie de l'onde impulsionnelle et le moyen pour calculer la pression sanguine maximale détermine, comme pression sanguine maximale, la pression du brassard sur la ligne droite de régression correspondant à une amplitude de l'onde impulsionnelle égale au deuxième niveau standard.

5. Tensiomètre électronique du type oscillométrique selon la revendication 3, où le second niveau standard est une amplitude de l'onde impulsionnelle égale à 70% de l'amplitude maximum vraie de l'onde impulsionnelle, et le moyen pour calculer la pression sanguine minimale détermine, comme pression sanguine minimale, la pression du brassard sur la ligne droite de régression correspondant à une amplitude de l'onde impulsionnelle égale au second niveau standard.
